Europäisches Patentamt

European Patent Office

Office européen des brevets

(19)

(11) Veröffentlichungsnummer: **0 161 686 B1**

## (12) EUROPÄISCHE PATENTSCHRIFT

(45) Veröffentlichungstag der Patentschrift: **13.02.91**

(51) Int. Cl.⁵: **A61M 1/14**

(21) Anmeldenummer: **85106027.7**

(22) Anmeldetag: **15.05.85**

(54) **Verfahren zum Füllen eines Blutschlauchsystems einer Hämodialysevorrichtung mit einer physiologischen Kochsalzlösung.**

(30) Priorität: **18.05.84 DE 3418434**

(43) Veröffentlichungstag der Anmeldung:
**21.11.85 Patentblatt 85/47**

(45) Bekanntmachung des Hinweises auf die
Patenterteilung:
**13.02.91 Patentblatt 91/07**

(84) Benannte Vertragsstaaten:
**DE FR GB IT**

(56) Entgegenhaltungen:
**EP-A- 0 104 897**
**EP-A- 0 122 604**
**DE-A- 3 237 016**

(73) Patentinhaber: **Fresenius AG**
**Gluckensteinweg 5**
**D-6380 Bad Homburg v.d.H.(DE)**

(72) Erfinder: **Polaschegg, Hans-Dietrich, Dr.**
**Grünwiesenweg 9**
**D-6370 Oberursel(DE)**

(74) Vertreter: **Dr. Fuchs, Dr. Luderschmidt**
**Dipl.-Phys. Seids, Dr. Mehler Patentanwälte**
**Abraham-Lincoln-Strasse 7**
**D-6200 Wiesbaden(DE)**

**Beschreibung**

Die Erfindung betrifft ein Verfahren zum Füllen der Blutseite, insbesondere eines Blutschlauchsystems und einer Blutkammer, einer Hämodialysevorrichtung mit physiologischer Elektrolytlösung, bei dem der Bluteingang und der Blutausgang eines Dialysators jeweils mit dem einen Ende eines Schlauchs verbunden sind und die anderen Enden der Schläuche mit einem Gefäß mit physiologischer Elektrolytlösung verbunden sind und die physiologische Elektrolytlösung in das Schlauchsystem und in die mit dem Schlauchsystem verbundene Kammer des Dialysators eingefüllt wird.

Vor der Hämodialyse, der Hämofiltration oder der Plasmafiltration, also vor dem Anschluß eines Dialysators an die Blutbahn eines Patienten, muß dieser Dialysator auf der Blutseite mit physiologischer Kochsalzlösung vollständig gefüllt werden, um diesen völlig von Luft zu befreien, da ansonsten die Gefahr besteht, daß Luft im Dialysator bleibt, was in der Folge zu einer Verminderung der Effektivität der Dialyse sowie zur Gerinnung von Blut im durch Luft verlegten Strömungspfad führt.

Zu diesem Zweck wird das Blutschlauchsystem an seinen Enden mit einem Gefäß mit physiologischer Kochsalzlösung verbunden, während die anderen Enden des Blutschlauchsystems mit dem Bluteingang und dem Blutausgang der vom Blut beaufschlagten Kammer des Dialysators verbunden sind.

Nach dem Anschluß des Systems an den vorteilhafterweise als Kochsalzlösungsbeutel ausgebildeten Behälter erfolgt eine komplizierte Manipulation des Dialysators, um die in der Blutkammer befindliche Luft durch die physiologische Kochsalzlösung zu verdrängen. Bei einem Hohlfaserdialysator, bei dem die Blutkammer aus einer Vielzahl von Kapillaren innerhalb der Hohlfäden gebildet wird, ist die Verdrängung der Luft durch den Durchflußwiderstand der Kapillaren schwierig, so daß die Füllung des Dialysators mit der physiologischen Kochsalzlösung sehr zeitaufwendig ist. Insofern muß der Dialysator mehrfach gewendet werden, ohne daß sichergestellt ist, daß er praktisch luftfrei ist.

Neben dem rein manuellen Verfahren zum Füllen des Blutschlauchsystems und des Dialysators mit einer physiologischen Kochsalzlösung wird bei einer bekannten Dialysevorrichtung die Drehrichtung der im Blutzuführungszweig angeordneten Blutpumpe mehrfach umgekehrt, so daß die physiologische Kochsalzlösung periodisch in und aus dem Dialysator gepumpt wird. Hierdurch wird zwar das manuelle Schütteln überflüssig, jedoch nicht völlig sichergestellt, daß sämtliche Bereiche der Blutkammer des Dialysators mit der physiologischen Kochsalzlösung gefüllt sind, d.h. diese Bereiche von Luft befreit sind. Dazu kommt noch, daß zusätzliche Sicherheitseinrichtungen vorgesehen werden müssen, die eine solche Drehrichtungsumkehr während der Dialyse im Fehlerfall sicher verhindern.

Aus der europäischen Patentschrift 104897 ist eine Single-Needle-Vorrichtung der eingangs erwähnten Art bekannt, bei der die Unterdruckeinheit mit einer im Blutschlauchsystem angeordneten Tropfkammer verbunden ist. Diese als Pumpe ausgebildete Unterdruckeinheit ist zur Regelung des in der Tropfkammer befindlichen Blutniveaus, nicht jedoch zum Einfüllen von physiologischer Elektrolytlösung in das Blutschlauchsystem vorgesehen.

Schließlich weisen die üblichen Hämodialysevorrichtungen, die im Single-Pass-Verfahren arbeiten, stromab des Dialysators im Dialysierflüssigkeitsweg eine Saugpumpe als Unterdruckeinheit auf, die zur Erzeugung des für die Ultrafiltration notwendigen Unterdrucks vorgesehen ist. Es ist jedoch bisher nicht bekannt, daß derartige Unterdruckpumpen zur Einfüllung der physiologischen Kochsalzlösung dienen.

Der Erfindung liegt daher die Aufgabe zugrunde, ein Verfahren zur Verfügung zu stellen, mit dem das Blutschlauchsystem und der Dialysator vor Beginn der Dialyse auf einfache Weise luftfrei mit physiologischer Kochsalzlösung gefüllt werden können.

Diese Aufgabe wird dadurch gelöst, daß man den Dialysator und das Blutschlauchsystem vor dem Einfüllen der physiologischen Elektrolytlösung gegenüber der Atmosphäre absperrt, anschließend mit einer Unterdruckeinheit evakuiert und die Elektrolytlösung in den evakuierten Bereich strömen läßt.

Dabei wird insbesondere der Dialysierflüssigkeitsweg stromauf und stromab des Dialysators unterbrochen, die mit dem Blutweg verbundene Dialysatorkammer wird evakuiert wobei der Blutweg stromauf und stromab des Dialysators verschlossen ist, anschließend wird der Verschluß des Blutwegs stromauf des Dialysators aufgehoben und die Kochsalzlösung in den evakuierten Bereich strömen gelassen.

Bei dem erfindungsgemäßen Verfahren wird die Evakuierung vorteilhafterweise mit Hilfe einer Unterdruckeinheit, insbesondere mit einer Vakuumpumpe vorgenommen, die mit dem Blutschlauchsystem verbunden ist.

Vorteilhafterweise ist die Vakuumpumpe mit einer Tropfkammer verbunden, die stromab des Dialysators im Blutabführungszweig angeordnet ist und die üblicherweise zur Entfernung von evtl. im Blut vorliegender Luft dient. An diese Tropfkammer ist die Vakuumpumpe über eine Leitung angeschlossen.

Des weiteren ist in den Blutzuführungszweig des Blutsystems eine Blutpumpe eingeschaltet, die üblicherweise als peristaltische Schlauchpumpe ausgebildet ist. Diese peristaltische Schlauchpumpe sperrt in der Ruhelage den Blutzuführungszweig des Blutsystems, so daß anstelle der Blutpumpe auch eine Klemme eingesetzt werden kann. In diesem Fall ist eine weitere Blutpumpe notwendig, die das Blut durch das Schlauchsystem und den Dialysator fördert.

Weiterhin ist im Blutabführungszweig vorteilhafterweise eine Klemme, insbesondere stromab der Tropfkammer vorgesehen, die in gleicher Weise wie die Blutpumpe den Blutschlauch sperrt.

Da die Membran des Dialysators bei Anlegen von Unterdruck luftdurchlässig ist, sind der Dialysierflüssigkeitseingang und -ausgang des Dialysators, insbesondere die mit diesem Eingang und diesem Ausgang des Dialysators verbundenen Schläuche des Dialysierflüssigkeitswegs mit Hilfe von Klemmen oder dgl. unterbrochen, um die Zufuhr von Luft durch diesen Eingang und Ausgang zu verhindern.

Als Unterdruckeinheit kann eine übliche Vakuumpumpe eingesetzt werden, die einen Unterdruck von 100 - 500 mbar abs. erzeugen kann. Dies kann eine übliche Wasserstrahlpumpe, eine Schlauchpumpe oder eine übliche Miniaturvakuumpumpe sein, die an die Tropfkammer angeschlossen ist. Vorteilhafterweise kann als Unterdruckeinheit die bei jedem Dialysegerät als Entgasungsvorrichtung vorgesehene Unterdruckpumpe, die üblicherweise einen Unterdruck von -0,8 bar erzeugt, verwendet werden.

Das erfindungsgemäße Verfahren wird auf folgende Weise durchgeführt:

Der Dialysierflüssigkeitseingang und -ausgang des Dialysators wird zunächst mit dem Schlauchsystem für die Zufuhr und Abfuhr der Dialysierflüssigkeit verbunden, wobei dieses Schlauchsystem durch Klemmen unterbunden wird. In alternativer Weise können der Eingang und der Ausgang des Dialysators für die Dialysierflüssigkeit mit je einem Stopfen verschlossen werden, wodurch der Dialysierflüssigkeitsweg stromauf und stromab des Dialysators ebenfalls unterbrochen oder verschlossen wird.

Anschließend werden die Blutanschlüsse des Dialysators mit dem Blutschlauchsystem verbunden, wobei der Blutzuführungsschlauch in die Blutpumpe eingelegt und das Ende mit dem Eingang des Dialysators verbunden wird. Andererseits wird der blutseitige Ausgang des Dialysators mit dem Blutabführungsschlauch verbunden, in den vorteilhafterweise eine Tropfkammer eingeschaltet ist. Die von der Tropfkammer abgehende Leitung wird anschließend in die vorstehend erwähnte Klemme eingelegt, die diesen Schlauch ebenso verschließt wie die Blutpumpe den Blutzuführungsschlauch.

Schließlich werden die beiden Enden des Schlauchsystems, also des Blutzuführungsschlauchs und des Blutabführungsschlauchs mit einem mit steriler physiologischer Kochsalzlösung gefüllten Beutel verbunden, wobei die beiden Schlauchenden mit ihren Kegelanschlüssen mit dem Beutel, z.B. Frekaflex-Beutel der Anmelderin, verbunden werden.

Nachdem der Dialysator sowie die mit ihm verbundenen Schlauchsysteme von der Atmosphäre abgeschlossen sind, wird die Vakuumpumpe betätigt, die den abgeschlossenen Bereich praktisch von Luft befreit. Sobald der eingestellte Unterdruck erreicht ist, wird die Blutpumpe eingeschaltet, die zunächst die in dem restlichen Schlauch befindliche Luft durch den Dialysator fördert, die durch die Vakuumpumpe entfernt wird. Anschließend wird Kochsalzlösung in den evakuierten Bereich gepumpt und füllt dort sämtliche evakuierte Bereiche, insbesondere die Blutkammer des Dialysators, die vorteilhafterweise aus den Kapillaren eines Hohlfaserbündels gebildet wird.

Nach der Füllung des vorteilhafterweise senkrecht angeordneten Dialysators, bei dem die Kochsalzlösung von unten her eingefüllt wird, wird der Blutabführungsschlauch und anschließend die Tropfkammer gefüllt. Diese Tropfkammer weist vorteilhafterweise die üblichen Füllstandssensoren auf, die bei Erreichen eines bestimmten Füllstandes durch die Kochsalzlösung die venöse Klemme öffnen. Hierdurch evtl. eindringende Luft wird ebenfalls durch die Vakuumpumpe solange entfernt, bis der Füllstand in der Tropfkammer wieder erreicht wird. Danach wird die noch im Blutabführungsschlauch verbliebene Luft durch die Kochsalzlösung verdrängt. Anschließend wird die Blutpumpe solange noch weiterbetrieben, bis sicher sämtliche Luftreste aus dem gesamten System entfernt sind, wobei die Füllstandssensoren die Vakuumpumpe nur dann einschalten, wenn der Füllpegel unter den gewünschten Stand gefallen ist, bzw. abschalten, wenn der Flüssigkeitspegel über den gewünschten Stand steigt.

Nach der Füllung des Blutschlauchsystems und des Dialysators mit der Kochsalzlösung wird die Vakuumpumpe abgetrennt, der Kochsalzbeutel zusammen mit den ggf. vorhandenen stopfenförmigen Verschlüssen entfernt und der Patient auf die übliche Weise an das Dialysesystem angeschlossen.

Die Unterdruckeinheit, die an die Tropfkammer angeschlossen ist, kann natürlich auch in einer weiteren Ausführungsform zur Regelung des Füllstands in der Tropfkammer eingesetzt werden, d.h. auch wenn das System mit Blut gefüllt ist, kann überschüssige Luft durch diese Vakuumpumpe aus der Tropfkammer entfernt werden, wobei die Füllstandssensoren in Verbindung mit einer Regeleinheit zur Steuerung der Pumpe herangezogen werden.

Bei Dialysatoren, insbesondere Kapillardialysatoren, deren Membranporen im Anlieferungszustand nicht durch ein Füllmedium, wie Glycerin, verstopft und deshalb luftdurchlässig sind, wird vorteilhafterweise folgendes, alternatives Verfahren eingesetzt:

Dialysator und Schlauchsystem werden - wie beschrieben - vorbereitet. Die Dialysierflüssigkeitsschläuche werden angeschlossen, wobei der Dialysierflüssigkeitsschlauch stromauf des Dialysators verschlossen wird. Dies ist einfach dadurch möglich, daß das üblicherweise vorhandene Bypass-Ventil in die Stellung "Bypass" geschaltet wird. Mit der bei Unterdruck geregelten oder Transmembrandruck geregelten Ultrafiltrationssteuerungen vorhandenen Unterdruckpumpe wird nun im Dialysierflüssigkeitsschlauch stromab des Dialysators ein Unterdruck erzeugt, wodurch die Luft aus dem Dialysator und dem Blutschlauchsystem entfernt wird. Sobald ein stationärer Unterdruck von etwa 0,8 bar erzeugt ist, wird - wie vorstehend beschrieben - die die Blutpumpe in Gang gesetzt und das gesamte System mit der Salzlösung gefüllt, wobei eine bestimmte Menge Flüssigkeit natürlich ultrafiltriert wird.

Eine solche Verfahrensweise ist insbesondere bei der Dialysevorrichtung gemäß DE-A 33 13 421 möglich, auf deren Offenbarung ausdrücklich Bezug genommen wird.

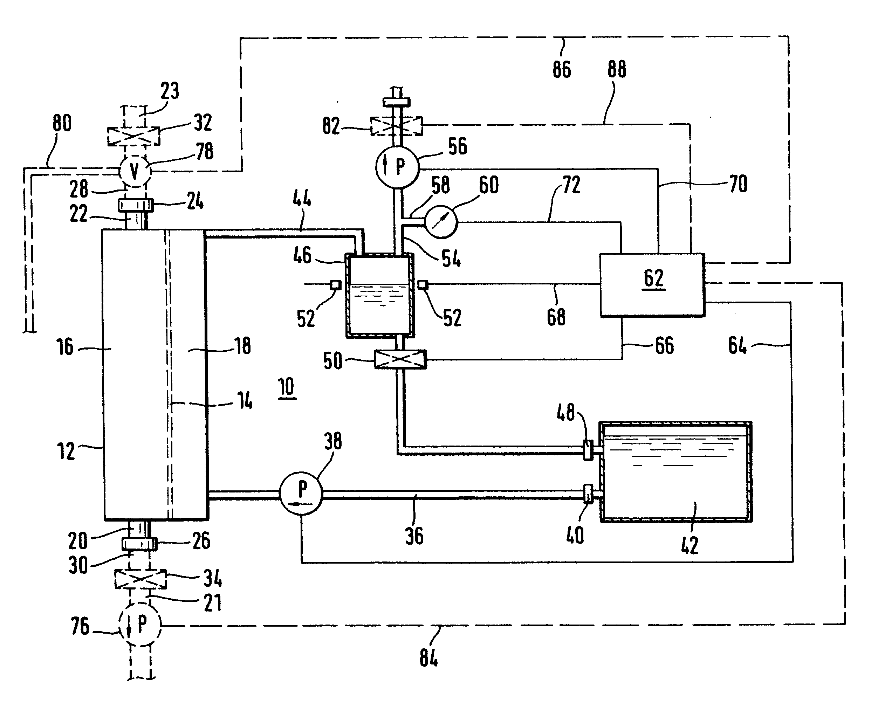

Weitere Einzelheiten, Merkmale und Vorteile sind aus der nachfolgenden Beschreibung eines Ausführungsbeispiels unter Bezugnahme auf die Zeichnung ersichtlich. Die Zeichnung zeigt schematisch zwei Ausführungsformen der erfindungsgemäßen Vorrichtung, mit denen das erfindungsgemäße Verfahren durchgeführt werden kann.

In der Figur ist die Dialysevorrichtung mit 10 bezeichnet. Sie weist einen Dialysator 12 auf, der als Hämodialysator, als Hämofiltrationseinheit oder als Plasmafilter ausgebildet sein kann. Dieser Dialysator 12 ist durch eine Membran 14 in eine Kammer 16, die von Dialysierflüssigkeit durchflossen wird, und eine Kammer 18 geteilt, die von Blut durchflossen wird. Die Enden der Kammer 14 weisen jeweils Anschlüsse 20 und 22 auf, die in einer ersten Ausführungsform mit stopfenartigen Verschlüssen 24 und 26 während der Füllphase des Dialysators 12 verschlossen sind. Anstelle dieser Verschlüsse 24 und 26 können jedoch auch die Anschlüsse 20 und 22 bereits mit einem gestrichelt gezeichneten Schlauchsystem für die Dialysierflüssigkeit, also mit einem Zuführungsschlauch 28 und einem Abführungsschlauch 30 verbunden sein. Diese Schläuche sind im Bereich des Eingangs und Ausgangs des Dialysators 12 mit Absperrorganen, insbesondere Klemmen 32 und 34 versehen, die die Schläuche 28 und 30 in der Füllphase von der Atmosphäre absperren. Als Absperrorgan kommt auch ein Bypassventil infrage.

Die blutseitige Kammer 18 ist an ihrem Eingang mit einem Blutzuführungsschlauch 36 verbunden, in den eine Blutpumpe 38 eingeschaltet ist. Das andere Ende des Blutzuführungsschlauchs 36 weist einen Kegelanschluß 40 auf, der mit einem Behälter 42 verbunden ist, der mit steriler physiologischer Kochsalzlösung gefüllt ist.

Der Auslaß der blutseitigen Kammer 18 des Dialysators 12 ist mit einem Blutabführungsschlauch 44 verbunden, in den eine Tropfkammer 46 eingeschaltet ist, die üblicherweise senkrecht angeordnet ist. Das Ende des Blutabführungsschlauchs ist bei der Zweinadelmethode ebenfalls mit einem Kegelanschluß 48 versehen, der wiederum mit dem Behälter 42 mit Kochsalzlösung verbunden ist.

Stromab der Tropfkammer 46 ist weiterhin am Blutabführungsschlauch ein Absperrorgan 50 vorgesehen, das vorteilhafterweise als Klemme ausgebildet ist.

Weiterhin ist am Außenumfang der Tropfkammer 46 eine Niveaudetektoreinheit 52 vorgesehen, mit der das Flüssigkeitsniveau in der Tropfkammer 46 erkannt und ein entsprechendes Signal abgegeben werden kann.

Von der Oberseite der Tropfkammer 46 erstreckt sich weiterhin eine Leitung 54, in die eine Unterdruckeinheit 56 eingeschaltet ist, die vorteilhafterweise als Vakuumpumpe ausgebildet ist. Von dieser Leitung kann in einer speziellen Ausführungsform eine weitere Leitung 58 abzweigen, die mit einem Druckmeßgerät 60 verbunden ist.

Die Dialysevorrichtung 10 weist weiterhin eine Regeleinheit 62 auf, die über die elektrische Leitung 64 mit der Blutpumpe 38, die elektrische Leitung 66 mit dem Absperrorgan 50, die elektrische Leitung 68 mit der Niveaudetektoreinheit 52 und mit der Leitung 70 mit der Unterdruckeinheit 56 verbunden ist. Ggf. kann die Regeleinheit 62 über eine Leitung 72 mit dem Druckmeßgerät 60 verbunden sein.

Mit dieser Regeleinheit 62 werden sämtliche Verfahrensabläufe während der Füllphase in der Dialysevorrichtung 10 geregelt.

Wie bereits vorstehend erläutert, betätigt die Regeleinheit 62 zunächst über die Leitung 70 die Vakuumpumpe. Nach Erreichen eines bestimmten Unterdrucks, der ggf. mit dem Druckmeßgerät 60 gemessen und an die Regeleinheit 62 abgegeben wird, wird über die Leitung 64 die Blutpumpe 38 betätigt, mit der physiologische Kochsalzlösung von unten her in die blutseitige Kammer 18 des Dialysators 12 gepumpt wird. Nach dem Füllen der Kammer 18 läuft die Kochsalzlösung in der Leitung 44 über und gelangt bis zur Klemme 50, die während dieser Phase des Füllvorgangs geschlossen gehalten wird. Sobald die Kochsalzlösung auf das Niveau der Niveaudetektoreinheit 52 angestiegen ist, wird ein Signal von dieser Einheit 52 über die Leitung 68 an die Regeleinheit 62 abgegeben, die die Klemme 50 öffnet und die Vakuumpumpe 56 gemäß dieser Ausführungsform ausschaltet. Dabei läuft die Blutpumpe 38 solange weiter, bis die gesamte Blutabführungs leitung 44 mit physiologischer Kochsalzlösung und die Tropfkammer 46 bis zur Höhe der Niveaudetektoren 52 gefüllt sind. Anschließend wird die Blutpumpe ausgeschaltet. Sollte der Füllstand jedoch nicht erreicht sein, so wird die Vakuumpumpe 56 über die Regeleinheit 62 eingeschaltet, die durch ihre Pumpwirkung den Füllstand in der Tropfkammer 46 hebt und hierdurch zugleich Kochsalzlösung aus dem Gefäß 42 ansaugt.

In einer weiteren Ausführungsform des erfindungsgemäßen Verfahrens wird die Blutpumpe 38 bei geschlossener Klemme solange betrieben, bis der Füllstand in der Tropfkammer 46 erreicht ist. Anschließend wird die Pumpe 38 abgestellt und die Klemme 50 geöffnet, wobei die Vakuumpumpe 56 bei dem gesamten Füllvorgang betrieben wird. Die überschüssige Luft in der Blutabführungsleitung stromab der Klemme 50 wird dabei durch die Wirkung der Pumpe 56 angesaugt, wobei zugleich Kochsalzlösung durch diese Leitung in die Tropfkammer 46 gesaugt wird. Ist diese Luft entfernt und der Füllstand in der Tropfkammer 46 erreicht, so schaltet die Regeleinheit 62 die Vakuumpumpe 56 aus und betreibt ggf. nochmals die Blutpumpe 38, um die Kochsalzlösung umzuwälzen.

Anschließend kann das Gerät wie üblich mit dem Patienten verbunden werden.

Zusätzlich zu dem beschriebenen Füllverfahren sind weitere Modifikationen möglich, bei denen zu Beginn ein Teil der Flüssigkeit verworfen wird, d.h. am Beginn des Füllens lediglich der arterielle Anschluß 36 des Blutschlauchsystems mit dem Kochsalzbehälter 42 verbunden wird.

Gemäß einer weiteren Ausführungsform kann die Regeleinheit 62 auch zur Regelung des Füllstandes in der Tropfkammer 46 mit Hilfe der Niveaudetektoreinheit 52 benutzt werden. Hierzu wird der Füllstand des Bluts in der Tropfkammer 46 mit Hilfe der Niveaudetektoreinheit 52 gemessen und die Vakuumpumpe 56 eingeschaltet, sobald der Blutpegel unter den unteren zulässigen Füllstand in der Tropfkammer 46 fällt. Durch die Entfernung der Luft aus der Tropfkammer 46 mit Hilfe der Vakuumpumpe 56 wird dann der Blutpegel wieder angehoben, was durch ein entsprechendes Signal der Niveaudetektoreinheit 52 zum Abschalten der Vakuumpumpe 56 durch die Regeleinheit 62 führt.

Falls jedoch dieser Pegel innerhalb einer vorbestimmten Zeit nicht erreicht wird, betätigt die Regeleinheit 62 die Klemme 50 und schaltet zugleich die gesamte Dialysevorrichtung 10 ab, da in diesem Fall vermutlich ein ernsthafter Fehler des Hämodialysesystems 10 vorliegt.

Durch Umkehr der Vakuumpumpe 56 kann der Spiegel in der Tropfkammer 46 auch gesenkt werden, wobei Luft in die Tropfkammer vorteilhafterweise über ein Schutzfilter 74 hineingepumpt wird. Dieses Schutzfilter 74 ist vorteilhafterweise stromab (d.h. bei dem üblichen Unterdruckbetrieb) der Vakuumpumpe 56 vorgesehen. Dies ist insbesondere dann notwendig, wenn durch ein kleines Leck im Bereich der Tropfkammer 46 und der Anschlüsse Luft entweicht.

In einer weiteren Ausführungsform wird der Dialysator 12 zur Herstellung eines Dialysierflüssigkeitswegs an seinen Anschlüssen 20 und 22 mit Schlauchleitungen 21 und 23 verbunden. In die als Ableitung dienende Leitung 21 ist dabei die eine als Unterdruckeinheit dienende Pumpe 76 eingeschal tet, die in Verbindung mit einem nicht gezeigten, stromauf des Dialysators angeordneten Strömungswiderstand bei der Dialyse den zur Ultrafiltration notwendigen Unterdruck erzeugt.

Stromauf des Dialysators kann die als Zuleitung dienende Leitung 23 wiederum mit der Klemme 32 versehen sein, die zum Füllen des Dialysators mit der physiologischen Elektrolytlösung (Kochsalzlösung) verschlossen wird. Vorteilhafterweise ist anstelle der Klemme 32 ein Ventil in der Zuleitung 23 vorgesehen, das insbesondere als Bypassventil 78 ausgebildet ist, von dem eine Bypassleitung 80 abzweigt. Zum Füllen des Blutschlauchsystems mit Kochsalzlösung ist dieses Bypassventil 78 in den Bypassbetrieb geschaltet, d.h. die Leitung 23 ist in Richtung auf den Dialysator 12 gesperrt.

Sofern die Tropfkammer 46 mit einer Entlüftungsleitung 54 versehen sein sollte, ist letztere mit einem Absperrorgan 82 versehen, die vorteilhafterweise als Klemme ausgebildet ist. Dieses Absperrorgan 82 läßt sich im Füllbetrieb unter Absperren der Leitung 54 schließen.

Wie in der Figur gezeigt, sind die Saugpumpe 76, das Bypassventil 78 und das Absperrorgan 82 über strichliert gezeigte elektrische Leitungen 84, 86 und 88 mit der Regeleinheit 62 verbunden, die diese -wie nachstehend beschrieben-entsprechend einem vorbestimmten Programm aktiviert.

Nachstehend ist die Funktion der zweiten Ausführungsform erläutert:

Das Blutschlauchsystem,bestehend aus den Blutschlauchleitungen 36 und 44 sowie der Tropfkammer 46, wird an seinen Enden 40 und 48 mit dem Behälter 42 verbunden, der die physiologische Kochsalzlösung aufweist.

Danach wird die Klemme 32 oder das Bypassventil 78 geschlossen oder aber es wird der Anschluss 22 mit einem Verschlußstopfen 24 verschlossen. Mit Hilfe der Regeleinheit 62 wird die stromab des Dialysators angeordnete Unterdruckeinheit 76 in Betrieb genommen mit der Folge, daß zunächst die Luft aus den beiden Kammern 16 und 18 des Dialysators 12 und danach aus dem Blutschlauchsystem entfernt wird. Wie bereits eingangs erläutert, wird die zweite Ausführungsform insbesondere bei solchen Membranen 14 eingesetzt, die im noch nicht benetzten Zustand luftdurchlässig sind.

Nach der Entfernung der Luft aus dem Dialysator und dem Blutschlauchsystem, was vorteilhafterweise mit Hilfe des Druckmeßgerätes 60 festgestellt werden kann, wird der Behälter 42 so positioniert, daß die Kochsalzlösung in die Leitung 36 abfließen kann. Danach werden mit Hilfe der Regeleinheit 62 -wie vorstehend beschrieben- die Blutpumpe 38 und die Klemme 50 in Betrieb genommen, wobei gegebenenfalls der Niveaudetektor 52 wiederum zu Regelzwecken eingesetzt wird. Nach dem völligen Füllen des Dialysators mit Kochsalzlösung wird die Klemme 82 geöffnet und es werden die Saugpumpe 76 und das Bypassventil 78 außer Betrieb gesetzt.

Schließlich kann -wie vorstehend ebenfalls erläutert- eine weitere Unterdruckeinheit 56 mit der Tropfkammer 46 verbunden sein, die zu diesem Zeitpunkt in Betrieb gesetzt wird, um eventuell noch im Blutschlauchsystem vorhandene Luft durch die Leitung 54 zu entfernen bzw. das Flüssigkeitsniveau in der Topfkammer 46 mit Hilfe der Niveaudetektoreinheit 52 und der Regeleinheit 62 zu regeln.

## Ansprüche

1. Verfahren zum Füllen der Blutseite einer Hämodialyservorrichtung mit einer physiologischen Elektrolytlösung, bei der der Bluteingang und der Blutausgang eines Dialysators (12) jeweils mit dem einen Ende jeweils eines Schlauchs (36,44) verbunden ist und die anderen Enden der Schläuche (36,44) mit einem Gefäß (42) mit physiologischer Elektrolytlösung verbunden sind, wobei die physiologische Elektrolytlösung in das Schlauchsystem und in die mit,dem Schlauchsystem verbundene Kammer des Dialysators (12) eingefüllt wird, **dadurch gekennzeichnet**, daß man den Dialysator (12) und das Blutschlauchsystem,- (36, 44, 46) vor dem Einfüllen der physiologischen Elektrolytlösung gegenüber der Atmosphäre absperrt, anschließend mit einer Unterdruckeinheit (56, 76) evakuiert und die Elektrolytlösung in den evakuierten Bereich strömen läßt.

## Claims

1. Method for filling the blood side of a hemodialysis device with a physiological electrolyte solution with the blood inlet and the blood outlet of a dialyzer (12) each being connected to one end of a tube (36, 44) and the other ends of the tubes ( 36, 44) being connected to a vessel (42) with physiological electrolyte solution, whereby the physiological electrolyte solution is introduced into the tubing system and into the chamber of the dialyzer (12) connected to the tubing system, characterized in that the dialyzer (12) and the blood tubing system (36, 44, 46) prior to the introduction of the physiological electrolyte solution are shut off with respect to the atmosphere, thereafter evacuated with a partial vacuum unit (56, 76) and thereafter the electrolyte solution is allowed to flow into the evacuated region.

## Revendications

1. Procédé de remplissage du côté sanguin d'un dispositif d'hémodialyse avec une solution d'électrolyte physiologique, dans lequel l'entrée du sang et la sortie du sang d'un dialyseur (12) sont reliées chacune à une extrémité d'un tuyau (36, 44) et les autres extrémités des tuyaux (36, 44) sont reliées à un récipient (42) contenant la solution d'électrolyte physiologique, cette dernière étant introduite dans la tuyauterie et dans la chambre du dialyseur (12), reliée à la tuyauterie, caractérisé en ce qu'on sépare de l'atmosphère le dialyseur (12) et la tuyauterie de sang (36, 44, 46), avant d'introduire la solution d'électrolyte physiologique, en ce qu'on y fait ensuite le vide avec une unité de dépression (56, 76) et en ce qu'on laisse arriver la solution d'électrolyte dans la zone où l'on a pratiqué le vide.